# EUROPEAN PATENT APPLICATION

(11) **EP 2 082 746 A1**
(43) Date of publication of application: **29.07.2009**
(21) Application number: 08100953.2
(22) Date of filing: 25.01.2008
(51) Int. Cl.: A61K 36/896, A61P 35/00

(54) **Extract from Urginea Maritima for use in the treatment of non small cell lung cancer (NSCLC) and other solid tumors**

(71) Applicant: Bozcuk, Hakan, Southport, QLD 4215 (AU); Özdogan, Mustafa, 07070 Antalya (TR)
(72) Inventor: Bozcuk, Hakan Gold Coast Hospital Department of Hematology/Oncology, Southport, Queensland 4215 (AU); Özdogan, Mustafa Akdeniz University Medical Faculty Division of Medical Oncology, 07070, Antalya (TR); Aykurt, Oktay Akdeniz University Medical Faculty Division of Medical Oncology, 07070, Antalya (TR); Topcuoglu, Fatih Akdeniz University Medical Faculty Division of Biology, 07070, Antalya (TR); Öztürk, Hasan Akdeniz University Medical Faculty Department of Biochemistry, 07070, Antalya (TR); Ekinci, Deniz Akdeniz University Medical Faculty Division of Medical Oncology, 07070, Antalya (TR); Karadeniz, Asuman Akdeniz University Medical Faculty Division of BiologyDivision of Biology Division of Biology, 07070, Antalya (TR); Burgucu, Durmus Akdeniz University MedicalDivision of Hematology Division of Hematology, 07070, Antalya (TR)
(74) Representative: Barz, Peter

(57) **Abstract**

Extracts from Urginea Maritima (Liliaceae) have been found useful in the treatment of Non Small Cell Lung Cancer (NSCLC) and other solid tumors.

## Description

### Background

Urginea Maritima (UM), Sea Bulb or White Squill, is a native plant found in the Mediterranean area, in North Africa and India. It belongs to the family of Liliaceae. The plant is known to exert diuretic and cardiotonic properties and has been used as a cough syrup (1).

Urginea Maritima is toxic and is known to cause human and animal poisoning (2, 3). There are also reports on an insecticide effect of Urginea Maritima (4).

In an article (7) published in 2001, the authors obtained 33 compounds from the bulbs of Urginea Maritima and referred to its use as a cardiotonic diuretic for the treatment of cardiac marasmus and edema.

### Summary of the Invention

The present invention is based on the surprising finding that extracts from Urginea Maritima are useful in the treatment of Non Small Cell Lung Cancer (NSCLC) and other solid tumors such as breast and colon cancer.

NSCLC is the leading cause of cancer-related deaths worldwide (5).Despite the advances in diagnosis and treatment of this disease, over the last 30 years the prognosis remained largely unchanged, and overall only 15% of patients with NSCLC are cured (6).

The inventors have explored the activity of Urginea Maritima extract in NSCLC cell culture and found it to be highly effective in the treatment of NSCLC and other solid tumors.

More specifically, methanol and water extracts from bulb and leaf parts of Urginea Maritima were prepared These extracts in low and high concentrations were added to a A549 NSCLC cell culture (16), either on their own or in combination with gemcitabine and cisplatin. Growth inhibition of the A549 cells caused by these treatments was tested using the MTT (3-(4,5-dimethylthiazol-2-yl)2,5-diphenyl tetrasodium bromide) assay (11,17). Additionally, an Annexin V-FITC apoptosis detection kit (Biovision Inc, Mountain View CA) was used to detect necrotic, early and late apoptotic activity after 72 hours of incubation.

As a result, the bulb extract was found to be more effective than cisplatin and gemcitabine (P<0.001, and P=0.097, respectively), currently the most potent drugs in the treatment of NSCLC The bulb extract was also better than leaf extract (P< 0.001) and methanol extraction yielded more cytotoxicity than water extraction By adding an antioxidant cocktail, the efficacy of the Urginea Maritima extract was improved from an inhibitory concentration 50% value (IC50) of 22 µg / ml to a value of 0.02 µg / ml,

Accordingly, the present invention also relates to pharmaceutical compositions comprising an extract from Urginea Maritima.

The pharmaceutical compositions can be used in all pharmaceutical forms, for instance, for oral administration in the form of coated tablets, tablets, capsules and solutions, and as injection preparations for intravenous or intramuscular injection For the manufacturing of such pharmaceutical preparations the optionally dried extracts can be formulated in the usual manner using excipients and carriers such as lactose, starch, microcrystalline cellulose, magnesium stearate and talc or water, alcohols, polyethylene glycols, glycerol esters. Various other additives may also be used such as antioxidants, preservatives, lubricants, wetting agents, emulsifiers, coloring agents, flavor correctives and flavoring agents. In a preferred embodiment, the pharmaceutical compositions comprise an antioxidant cocktail consisting of mannitol, sodium ascorbate and ascorbic acid For improved stability, the extracts and pharmaceutical compositions may be stored under nitrogen protection.

In another aspect, the present invention relates to a method for treating Non Small Cell Lung Cancer (NSCLC) and other solid tumors such as breast and colon cancer by administering to a patient in need of such treatment an effective amount of an extract from Urginea Maritima or a pharmaceutical composition containing such extract

The extracts and pharmaceutical compositions according to the present invention may be administered in combination with one or more other anticancer drugs such as cisplatin or gemcitabine

The following detailed description is intended to further illustrate the present invention without however limiting it.

### MATERIAL AND METHODS

### 1. Extract Preparation

For the purpose of extract preparation, only Urginea Maritima plants far away from roads were utilized, Any environmental exposure to pesticides was thus excluded,

The methanol and water extracts were prepared as described in the literature (8) using 15 g of plant and 60 g of solvent, then evaporated to dryness at 65°C with a rotary evaporator and stored at -20°C with no exposure to light Separate extracts were prepared from the bulb and leaf parts of the plant.

### 2. Cell line and treatment of cells

An A549 NSCLC cell line (16) was used for the cytotoxicity assays. Depending on the nature of the experiments, various treatments were applied to the cell line.

In the first part of the experiments where the aim was to determine the effect of the type of extract (leaf versus bulb), the following treatments were carried out:
1) Gemcitabine 1 nm / ml
2) Cisplatin 1 µg / ml
3) Extract (bulb or leaf) 1 µg / ml (low dose)
4) Extract (bulb or leaf) 100 µg / ml (high dose)
5) Gemcitabine 1 nm / ml + Extract (bulb or leaf) 1 µg / ml (low dose)
6) Gemcitabine 1 nm / ml + Extract (bulb or leaf) 100 µg / ml
7) Cisplatin 1 µg / ml + Extract (bulb or leaf) 1 µg / ml
8) Cisplatin 1 µg / ml + Extract (bulb or leaf) 100 µg / ml
9) Control (no treatment)

In the second part of the experiments where the intention was to increase the activity of the extract, the following treatments were carried out:
1) Aged bulb extract (fresh extract kept for one month, used as control), 1, 0.1, 0.01 µg / ml
2) N2 modified bulb extract, 1, 0.1, 0 01 µg / ml
3) Antioxidant cocktail modified bulb extract, 1, 0.1, 0.01 µg / ml

The concentrations of gemcitabine and cisplatin as used in this study were chosen as it was previously shown that these concentrations were close to the IC50 values of these drugs in this cell line (9,10). The concentrations employed for the extract ranged between 100 and 0.01 µg / ml

### 3. MTT assay and cell culture

Growth inhibition of the A549 cells by the above treatments was tested by the MTT (3-(4,5-dimethylthiazol-2-yl)-2,5diphenyl tetrasodium bromide) assay, as described in the literature (11), and as we had used it in our previous work (9,12).

The MTT assay was used to indicate the level of cytotoxicity obtained by a specific treatment with reference to control cells (without any treatment) The level of absorbance, as assessed by spectrophotometry showed the amount of relative cytotoxicity Briefly, cancer cells were seeded into 96-well microtiter plates at appropriate densities. After successful seeding, cells were exposed to various treatments as mentioned above for 72 hours. Tests were repeated 5 times. At the end of treatment, 20 µL of 5 mg / ml MTT (Sigma, St. Louis, MO, USA) was added to each well and the plates were incubated for 4 hours at 37°C. At the end, DMSO (150 µL) was added to each well and the optical absorbance at 570 nm was read on a plate reader Cytotoxicity was defined as "100 x (1 - [(absorbance of treated cells) / (absorbance of control cells)]),

### 4. Assessment of apoptosis

In line with the manufacturer's guidelines, an Annexin V-FITC apoptosis detection kit (Biovision Inc, Mountain View CA) was used to detect necrotic, early and late apoptotic activity after 72 hours of incubation After various treatments, cells (1 X 10⁶) were collected and resuspended in binding buffer, and Annexin V-FITC and propidium iodide were added to each sample and incubated in the dark for 5 minutes. Annexin V-FITC binding was determined by flow cytometry (Ex = 488 nm; Em = 530 nm) using a FITC signal detector (FL1) and propidium staining by the phycoerythrin emission signal detector (FL2)

### 5. Extract modification

In an attempt to augment the cytotoxicity of the bulb extract, two modifications were made

The first one was to place the extract under N2 gas protection in order to impede the oxidative damage to compounds of the extract

The second modification consisted of the addition an antioxidant cocktail made up of three compounds: Mannitol (250 parts), sodium ascorbate (25 parts), and ascorbic acid (1 part), with reference to weight.

### 6. Statistical Analysis

The magnitude of cytotoxicity with regard to different kinds of treatment, in relation to type of extract (bulb or leaf) was compared in a general linear model, utilizing the Bonferonni procedure as a post hoc test. In addition, to investigate the effect of modifications on efficacy of the bulb extract, the cytotoxicity induced was again compared in a multivariate model to test together the effects of type of modification, concentration of the extract, and their interaction term, on the magnitude of cytotoxicity. A P value < 0.05 was considered to be significant SPSS 15.0.0 (SPSS Inc , Chicago, IL, USA, release 15.0.0) was used for the statistical analysis.

### RESULTS

### 1. The effect of treatment and type of extract on cytotoxicity

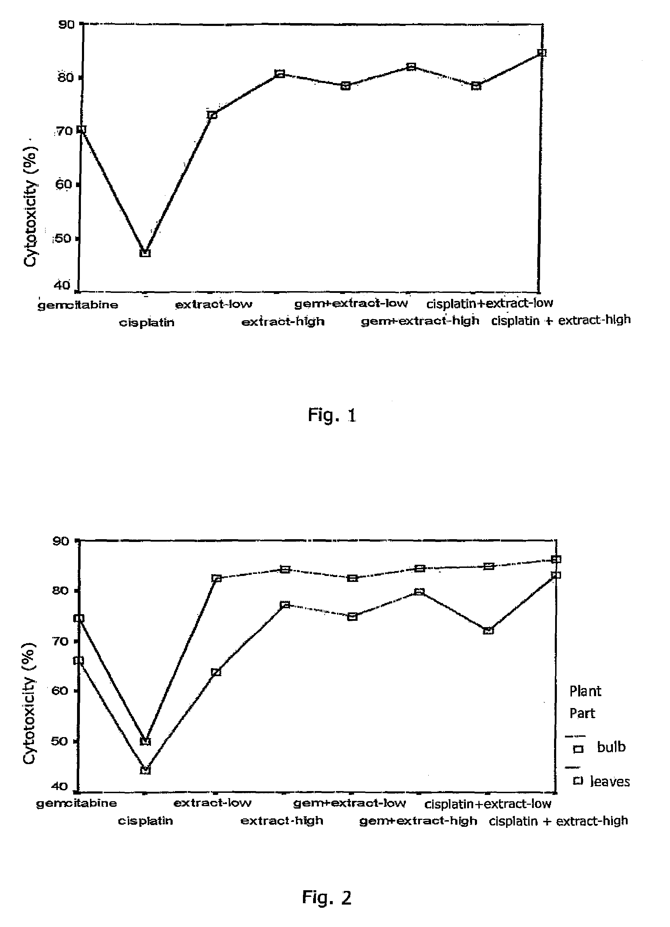

The treatments have yielded cytotoxicity between around 40% and 80%. Extract in low dose was as effective as gemcitabine, and more effective than cisplatin, but less effective than extract in high dose, with P values of 1.000, < 0.001 and < 0.001, respectively. See Table 1 and Figure 1 for details

**Table 1**

| Treatment | Cytotoxicity (%) | | 95% Confidence Interval | |
|---|---|---|---|---|
| | Mean | Std. Error | Lower Bound | Upper Bound |
| Gemcitabine | 70,332 | 1,155 | 68,044 | 72,620 |
| Cisplatin | 47,187 | 1,155 | 44,899 | 49,476 |
| Extract-low | 73,216 | 1,155 | 70,927 | 75,504 |
| Extract-high | 80,751 | 1,155 | 78,463 | 83,039 |
| Gem+Extract-low | 78,620 | 1,155 | 76,332 | 80,908 |
| Gem+Extract-high | 82,154 | 1,155 | 79,866 | 84,442 |
| Cisplatin+Extract-low | 78,504 | 1,155 | 76,216 | 80,792 |
| Cisplatin+Extract-high | 84,680 | 1,155 | 82,392 | 86,968 |

When only the type of extract is considered, extract derived from the bulb caused higher cytotoxicity than that from the leaves with a difference of approx. 8.5% (P < 0.001). See Table 2 and Figure 2 for details.

**Table 2**

| Treatment | Plant Part | Cytotoxicity (%) | | |
|---|---|---|---|---|
| | | Mean | Standard Deviation | N |
| Gemcitabine | bulb | 74,4590 | 9,5909 | 8 |
| | leaf | 66,2045 | 4,7832 | 8 |
| | Total | 70,3317 | 8,4719 | |
| Cisplatin | bulb | 50,0786 | 8,9319 | 8 |
| | leaf | 44,2962 | 5,1802 | 8 |
| | Total | 47,1874 | 7,6596 | 16 |
| Extract-low | bulb | 82,5691 | 2,9256 | 8 |
| | leaf | 63,8620 | 2,5860 | 8 |
| | Total | 73,2156 | 10,0218 | 16 |
| Extract-high | bulb | 84,2055 | 2,7331 | 8 |
| | leaf | 77,2969 | 3,2950 | 8 |
| | Total | 80,7512 | 4,6130 | 16 |
| Gem+Extract-low | bulb | 82,4429 | 2,7947 | 8 |
| | leaf | 74,7969 | 2,7047 | 8 |
| | Total | 78,6199 | 4,7590 | 16 |
| Gem+Extract-high | bulb | 84,5492 | 4,7306 | 8 |
| | leaf | 79,7584 | 1,2644 | 8 |
| | Total | 82,1538 | 4,1605 | 16 |
| Cisplatin+Extract-low | bulb | 84,8305 | 2,1417 | 8 |
| | leaf | 72,1779 | 2,7900 | 8 |
| | Total | 78,5042 | 6,9616 | 16 |
| Cisplatin+Extract-high | bulb | 86,3062 | 1,8214 | 8 |
| | leaf | 83,0532 | 5,6177 | 8 |
| | Total | 84,6797 | 4,3700 | 16 |
| Total | bulb | 78,6801 | 12,4659 | 64 |
| | leaf | 70,1808 | 12,1378 | 64 |
| | Total | 74,4304 | 12,9758 | 128 |

It has also been found that methanol extraction was more effective than water extraction in terms of cytotoxicity. Of course, other C1-C4 alcohols such as ethanol, n-propanol, isopropanol, n-butanol, sec-butanol and tert-butanol may be used instead of methanol.

### 2 Extract modification

In order to counteract the decline in activity of the extract aver time, two modifications were made: (1) the addition of N2 gas and (2) the incorporation of an antioxidant cocktail into the extract The activity was enhanced particularly with the addition of the antioxidant cocktail. See Table 3 for the cytotoxicity caused by different concentrations Specifically, the IC50 values for the plain extract, the N2-modified extract and the antioxidant cocktail-modified extract were around 0.22, 0.05 and 0.02 µg / ml, respectively.

**Table 3**

| Concentration | Type of Extract | Cytotoxicity (%) | | 95% Confidence Interval | |
|---|---|---|---|---|---|
| | | Mean | Std. Error | Lower Bound | Upper Bound |
| 1 µg / ml | aged only | 74,9 | 3,3 | 68,4 | 81,5 |
| | aged w/N2-1 µg | 82,4 | 3,3 | 75,9 | 88,9 |
| | aged w/cocktail-1 µg | 76,8 | 3,3 | 70,2 | 83,3 |
| 0,1 µg/ml | aged only | 29,6 | 3,3 | 23,0 | 36,1 |
| | aged w/N2-1 µg | 58,3 | 3,3 | 51,8 | 64,9 |
| | aged w/cocktail-1 µg | 72,5 | 3,3 | 66,0 | 79,1 |
| 0,01 µg/ml | aged only | 12,7 | 3,3 | 6,2 | 19,2 |
| | aged w/N2-1 µg | 25,3 | 3,3 | 18,8 | 31,9 |
| | aged w/cocktail-1 µg | 45,0 | 3,3 | 38,4 | 51,5 |

### 3. Mechanism of cytotoxicity

Flow cytometric analysis using Annexin V revealed that the major mechanism of cytotoxicity was via the augmentation of apoptosis. Leaf extract in low and high concentrations demonstrated apoptosis in 80% and 87.8% of cells, whereas bulb extract in low and high concentrations caused apoptosis in 82.6% and 93.4%, respectively.

### References

1. Drugstore Museum : www.drugstoremuseum.com/sections/level_info2.php? level_id=91&level=2
2. Tunckok Y, Kozan O, Cavdar C, et al. Urginea Maritima (Squill) tonicity. J Toxicol Clin Toxicol, 33 (1): 83-6, 1995.
3. El Bahri L, Djegham M, Makhlouf M Urginea maritima L (Squill): a poisonous plant of North Africa. Vet Hum Toxicol, 42 (2): 108-10, 2000.
4. AAIC 2000 Program. www.aaic.org/01progrm2.htm
5. Peto R, Chen ZM, Boreham J. Tobacco-the growing epidemic. Nat Med 1999;5:15.
6. Shrump DS, Nasser KA, Henscke C, et al Non-Small Cell Lung Cancer. In Cancer, Principles and Practice of Oncology, Lippincott Williams & Wilkins, Philadelphia 2005; 753-810. 7 lizuka M, Warashina T, Noro T. Bufadienolides and a new lignin from the bulbs of Urginea Maritima. Chem Pharm Bull, 49 (3): 282-6, 2001.
7. lizuka M, Warashina T, Noro T. Bufadienolides and a new lignin from the bulbs of Urginea Maritima. Chem Pharm Bull, 49 (3): 282-6, 2001.
8. Benkeblia N. Free radical scavenging capacity and antioxidant properties of some selected bulbs and garlic extracts. Brazilian Archives of Biology and Technology, 48 (5): 753-9, 2005.
9. Ozturk OH, Bozcuk H, Burgucu D, et al. Cisplatin cytotoxicity is enhanced with zoledronic acid in A549 lung cancer cell line: preliminary results of an in vitro study. Cell Biol Int, 31 (9): 1069-71, 2007.
10. Yang Y, LC QJ, Du QY, et al. Combined effects of cantide and chemotherapeutic drugs on inhibition of tumor cells' growth in vitro and in vivo. World J Gastroenterol, 11 (16): 2491-6, 2005.
11. Cui YY, Xie H, Qi KB, et al. Effects of Pinus Massoniata bark extract on cell proliferation and apoptosis of human Hepatoma BEL-7402 cells. World J Gastroenterol, 11(34): 5277-82, 2005.
12. Yildiz M, Celik-Ozenci C, Akan S, et al. Zoledronic acid is synergistic with Vinblastine to induce apoptosis in a multidrug resistance protein-1 dependant way Cell Biol Int, 30 (3), 278-82, 2006
13. Mekhail T, Kaur H, Ganapathi R, et al. Phase 1 trial of Anvirzel in patients with refractory solid tumours; Invest New Drugs, 24 (5): 423-7, 2006.
14. Pujol JL, Barlesi F, Daures JP. Should chemotherapy combination for advanced non-small cell lung cancer be platinum based? A meta-analysis of phase 3 randomised trials. Lung Cancer, 51 (3): 335-45, 2006.
15. Le Chevalier T, Scagliotti G, Natale R, et al. Efficacy of gemcitabine plus platinum chemotherapy compared with other platinum containing regimens in advanced non-small cell lung cancer: a metaanalysis of survival outcomes. Lung Cancer, 47 (1): 69-80, 2005.
16. Lieber M, Smith B, Szakal A, et al. A continuous tumor cell-line from a human lung carcinoma with properties of type 2 alveolar epithelial cells. Int J Cancer, 17 81): 62-70, 1976.
17. Kim J, Lee SK, Hwang ES, Kim JS, Kim K, Lee JH, Limited cytotoxic effect of adenoviral mediated p53 gene transfer in variable NSCLC cell lines, J Korean Cancer 1997; 29:565-75

## Claims

1. An extract from Urginea Maritima for use in the treatment of Non Small Cell Lung Cancer (NSCLC) and other solid tumors.

2. The extract of claim 1 obtained from the bulbs and/or the leaves of Urginea Maritima, preferably the bulbs thereof

3. The extract of claim 1 obtained by extraction with water or C1-C4 alcohols.

4. The extract of claim 3 obtained by extraction with methanol.

5. A pharmaceutical composition comprising an extract from Urginea Maritima according to any one of claims 1 to 4.

6. A pharmaceutical composition according to claim 5, additionally comprising one or more antioxidants.

7. A pharmaceutical composition according to claim 5 or 6 for use in the treatment of Non Small Cell Lung Cancer (NSCLC) and other solid tumors.

8. A method for treating Non Small Cell Lung Cancer (NSCLC) and other solid tumors by administering to a patient in need of such treatment an effective amount of an extract from Urginea Maritima according to any one of claims 1 to 4 or a pharmaceutical composition according to claim 5 or 6.

9. A method according to claim 8 which comprises additionally administering another anticancer drug such as cisplatin or gemcitabine
